# EUROPEAN PATENT SPECIFICATION

(11) **EP 1 709 144 B1**
(45) Date of publication and mention of the grant of the patent: **17.11.2010**
(21) Application number: 04806281.4
(22) Date of filing: 29.12.2004
(51) Int. Cl.: C11C 3/14, C09F 7/08

(54) **METHOD FOR THE PREPARATION OF CONJUGATED LINOLEIC ACID**
VERFAHREN ZUR HERSTELLUNG VON KONJUGIERTER LINOLENSÄURE
PROCEDE DE PREPARATION D'ACIDE LINOLEIQUE CONJUGUE

(30) Priority: 30.12.2003 HU 0304112
(43) Date of publication of application: 11.10.2006
(73) Proprietor: Bunge Növényolajipari Zártköruen Muködo Részvénytársasag, 1134 Budapest (HU)
(72) Inventor: DIANÓCZKI, Csilla, H-2310 Szigetszentmiklós (HU); KÖVÁRI, Józsefné, H-1162 Budapest (HU); NOVÁK, Lajos, H-1126 Budapest (HU); POPPE, László, H-1188 Budapest (HU); RECSEG, Katalin, H-1188 Budapest (HU)
(74) Representative: Szentpéteri, Adam
(86) International application number: PCT/HU2004/000124
(87) International publication number: WO 2005/063956

(56) References cited:
- EP-A- 0 902 082
- WO-A-01/51597
- US-A- 3 162 658
- US-A- 6 015 833
- "Fatty acid profiles of 80 vegetable oils with regard to their nutritional potential" EUR. J. LIPID. SCI. TECHNOL., vol. 109, 2007, pages 710-732,

## Description

### TECHNICAL FIELD

The present invention relates to a method for the preparation of conjugated linoleic acid from linoleic acid-containing materials by alkali isomerization.

### BACKGROUND ART

In the last years polyunsaturated conjugated fatty acids, particularly conjugated linoleic acid (CLA) gained growing interest. Polyunsaturated fatty acids have a carbon chain of 18 or more carbon atoms. Linoleic acid (c9,c12-octadecadienoic acid), linolenic acid and arachidonic acid belong to this group as essential fatty acids. These fatty acids contain isolated (not conjugated) double bounds of *cis* configuration. Though conjugated double bonds are rarely present in fatty acids, conjugated linoleic acid can be found in nature.

Conjugated linoleic acid was discovered at the beginning of the 1930s and in 1979 came into the limelight. The term "conjugated linoleic acid" includes positional and geometric isomers of linoleic acid, in which the double bonds are present in conjugated position, contrary to the naturally occurring polyunsaturated fatty acids, wherein the double bonds are isolated. As for their positions, the pair of double bounds may be in 7,9-, 8,10-, ... 12,14-positions, and they may have *cis-cis (c,c), cis-trans* (*c,t*) and *trans-trans* (*t,t*) geometry. Consequently, according to the theoretical possibilities, many CLA isomers may exist.

CLA naturally occurs in the meat of ruminants, in milk and diary products (cheese, butter, etc.), and can also be found in mother's milk. The isomers of the most significant biological activity are *c*9,*t*11- and *t*10,*c*12-octadecadienoic acids (Pariza M.W.; in Yurawecz M.P.; Mossoba, M.M.; Kramer, J.K.; Pariza, M.W.; Nelson, G.J. (Editors) Advances in Conjugated Linoleic acid Research, Volume 1, AOCS Press Champaign, IL, 1999, pp. 12-20).

The *c*9,*t*11-CLA isomer's natural forming site is the digesting system of ruminants, where it forms as an intermediate in the biohydrogenation of linoleic acid.

The biological activity of conjugated linoleic acid is complex and diverse. Many clinical and animal studies (both *in vivo* and *in vitro*) confirm the beneficial effect of CLA.

By studying the anticarcinogen properties of CLA on different cancerous cells, it was found that CLA reduces the chance of the development of different cancerous diseases.

In addition to its effect on cancerous diseases, CLA reduces the blood LDL cholesterol level and the chance of the development of coronary diseases, and it influences the immune system too.

Beside the effects mentioned above, conjugated linoleic acid also influences the lipid/protein metabolism. CLA improves feed conversion. Mixed into animal feed it promotes the increase of protein mass, thereby it may play significant role in animal feeding. Studies carried out with cows in milk showed significant decrease in the fat content of the milk and increase in the CLA content of the milk fat, whereas the milk production, feed uptake and milk protein content remained unchanged. These effects were observed when a mixture of the two biologically active CLA isomers (*c*9,*t*11 and *t*10,*c*12) or pure *t*10,*c*12-CLA isomer was added to the feed. In case of supplementation with *c*9,*t*11 isomer no significant positive changes were found.

In most of the earlier methods known for the preparation of CLA the main source of linoleic acid is sunflower, safflower or grape seed oil.

In some methods conjugated linoleic acid is produced by dehydration of castor oil. Though the starting material used in these methods is inexpensive, the reagents used make the method very expensive. The dehydration method is difficult to control, thus the product obtained this way is not competitive regarding its price and isomer composition.

In general, for the production of conjugated linoleic acid the known methods use alkali isomerization of linoleic acid-containing materials in different solvents, under different reaction conditions.

During alkali isomerization the solution of the linoleic acid-containing material is treated with a base at desired temperature. By acid treatment of the salt formed, and by working up the reaction mixture, conjugated linoleic acid is obtained, which is a mixture of different isomers.

The most advantageous solvent would be water, but the base strength of bases used for the isomerization is the lowest in aqueous medium, which necessitates the use of rather harsh reaction conditions (high pressure, very high temperature). Such an example can be found in US 6 015 833. In this case isomerization takes place under high pressure and at high temperature (180-210 °C) during a long (6-22.5 hours) reaction time, which impairs economic efficiency. In the product in addition to the two most important isomers many other conjugated linoleic acid isomers can be found, and the proportion of *t*,*t*-isomers is particularly high.

In US 4 164 505 a continuos flow reactor is used for carrying out the isomerization. A mixture of free fatty acids and aqueous sodium hydroxide solution is mixed at 80 °C, then the fatty acid soap formed is introduced into the reactor at 250-300 °C, where the isomerization takes place under high pressure and during short residence time (25 minutes). In spite of the relatively short reaction time many different isomers are formed, and in the product the quantity of *t*,*t* conjugated linoleic acid is especially high.

Two earlier patent specifications disclose different possibilities for the isomerization of fatty acids containing double bonds. According to US 2 343 644, isomerization is carried out with metal hydroxide bases in different glycols at about 200 °C. In US 2 242 230 different base/solvent combinations (NaOEt/EtOH, NaOBu/BuOH, Na/BuOH, NaOiPr/iPrOH, NaOtBu/tBuOH, KOH/MeOH, KOH/EtOH, KOH/BuOH, KOH/glycol, KOH/isoamyl alcohol, Et₃N/EtOH) were studied at a wide range of temperatures (100-195 °C according to the boiling point of the solvent used). The reaction time also varied within broad limits (5-100 hours), but in general it was about 24 hours. In case of reactions taking place at relatively low temperatures, the reaction time was rather long (at least 24 hours), while in case of reactions carried out at higher temperatures, although the reaction time was shorter (5-10 hours), the reaction conditions used resulted in significant impairment of the quality of the product. In addition, the strength and quantity of the base used influenced the composition of the product, as well as the necessary reaction time. Use of lower alcohols does not allow the use of sufficiently high temperature at atmospheric pressure, and in these solvents the base strength is also low. Therefore, a rather long reaction time should be used.

According to the method described in WO 01/18161, the alkali isomerization of vegetable oils is carried out with alkali metal hydroxides in the presence of excess amount of free glycerol, at high temperature (200-250 °C). Oils of high linoleic acid content or 90 % linoleic acid methyl ester are used as starting material, and the base is used in an excess of 1-50 %. To the soap formed glycerol is added at 100 °C, and the mixture is allowed to react for 4 hours at 230 °C. Then the reaction mixture is cooled and treated with acid, the product is washed with water and dried. The product, due to the reaction conditions used, contains many isomers. Though glycerol is a biocompatible solvent, because of the low base strength obtainable in this solvent, the reaction can be accomplished at high temperature, only, which facilitates the formation of non-desired isomers (first of all *t*,*t* isomers).

In the method described in WO 97/46230 1,2-propylene glycol (propane-1,2-diol) solvent and potassium hydroxide are used at 180 °C for performing isomerization. The product, due to the high reaction temperature, contains high amount of other CLA isomers in addition to the two biologically active ones.

Also 1,2-propylene glycol is used as solvent according to US 6 160 140, but the isomerization is carried out in the presence of lower amount of base (KOH or NaOH). Because of the lower reaction temperature (135 °C) mainly the two most important conjugated linoleic acid isomers are formed, but in spite of the rather long reaction time (47 hours) the conversation is relatively low (60-90 %), which is not favourable at all from the point of view of industrial applicability.

In the method disclosed in US 6 015 833, which starts from oil, an alkali metal hydroxide is used as base in 1,2-propylene glycol solvent at 130-170 °C, in particular at 150 °C, and the reaction time is 2-6.5 hours (at 150 °C 3.5 hours). The isomerization is at least 90 %, but it can be as high as 99.5 %. At 50 °C water, then hydrochloric acid is added to the reaction mixture. Propylene glycol goes into the aqueous phase, the traces of the solvent are removed in vacuum, and the final product is purified by further distillation. According to the data given in the patent specification, mainly the two biologically most important isomers (*c*9,*t*11-18:2 and *t*10,*c*12-18:2) are formed, with only a few % of other isomers. At higher temperature (210 °C) a shorter reaction time is enough to perform the conjugation, but in this case the product is a mixture of many isomers. Moreover the quantity of *t*,*t*-CLA significantly increases. The disadvantage of the method is that it requires a lot of water to remove the not perfectly biocompatible organic solvent. This way a great amount of waste water is formed, which is not favourable regarding environmental protection, and the regeneration of the solvent is also very expensive. A further disadvantage of the method is that the glycols (1,2-propylene glycol, 1,2-ethylene glycol) are not perfectly accepted in food industry.

In US 3 162 658 a process for catalytic isomerization of esters or amides of unconjugated polyethenoid fatty acids to conjugated isomers thereof using catalytic amount of alkali metal alkoholates as base (0.05 to 5 wt%) is described.

The isomerization method discussed in WO 00/18944 is almost the same as the one described above (US Pat. No. 6 015 833), but the free acid formed is reacted with glycerol in the presence of an enzyme catalyst to give glycerides. During the isomerization the two biologically most active isomers (*c*9,*t*11-18:2 and *t*10,*c*12-. 18:2) are formed in the highest quantity in nearly the same ratio.

### DISCLOSURE OF THE INVENTION

The object of the present invention is to develop a new method for the preparation of conjugated linoleic acid by alkali isomerization, which does not have the disadvantages of the known methods. Accordingly, our aim was to work out a method, which results essentially in the desired isomers and the isomerization takes place with high efficiency. A preferred method is also favourable from environmental point of view, moreover, it uses raw materials, for example solvent, which are completely accepted in food industry.

The invention is based on the recognition that the above objects can be achieved if the alkali isomerization method is carried out in apolar solvent. In apolar solvent the base strength is increased, which allows the use of milder reaction conditions. Accordingly, the invention provides two method variations for the preparation of conjugated fatty acids from linoleic acid-containing materials by alkali isomerization, wherein apolar solvent is used as solvent.

In the method variation I the alkali isomerization reaction, wherein a linoleic acid-containing material is treated with a base in solution, is carried out in an apolar solvent or in a mixture of apolar solvents using an alkali metal alcoholate as base. In the method variation II a long chain alcohol of 6-20 carbon atoms or a mixture of long chain alcohols of 6-20 carbon atoms is used as solvent, and an alkali metal hydroxide or an alkali earth metal hydroxide or an alkali metal alcoholate is used as base, at moderate (100-170 °C) temperature.

The method variations of the invention are defined in claim 1 and claim 15, respectively.

### MODES FOR CARRYING OUT THE INVENTION

In the methods according to the invention preferably materials of high linoleic acid content (where the linoleic acid content is generally higher than 50 %) are used, for example oils (sunflower, safflower, soybean, corn oils) or a mixture thereof, mixtures of free fatty acids obtainable from these oils, as well as by-products forming during refining of vegetable oils, such as deodorization distillate containing free fatty acids in relatively high quantity.

According to a preferred embodiment of the invention, in method variation I the isomerization reaction is carried out in the presence of a phase transfer catalyst.

In method variation I in the alkali isomerization reaction preferably an acyclic or cyclic alkane, such as n-hexane, n-heptane, n-octane, isooctane, cyclohexane, cycloheptane, cyclooctane etc. is used as apolar aprotic solvent, the volume of which is preferably 2-100 times higher than that of the oil. Other apolar aprotic solvents, such as benzene, toluene, ethers, petroleum ether, long chain alcohols, etc. can also be used. Also a mixture of apolar aprotic solvents can be used as a solvent.

The most preferred bases used according to the invention are alkali metal alcoholates of secondary and tertiary alcohols because of their high base strength.

As phase transfer catalyst for example, quaternary ammonium salts (such as tetrabutylammonium chloride (TBACI), tetrabutylammonium hydrogensulphate (TBAHSO₄), etc.) or alkali metal salts of fatty acids (for example potassium oleate, potassium linoleate etc.) or other hydrophohic anionic or cationic compounds are used. The quantity of the phase transfer catalyst is preferably 0.1-10 %.

In method variation I the isomerization is carried out preferably at a temperature ranging from 20 °C to the boiling point of the solvent, to a maximum of 140 °C, more preferably at 55 to 70 °C.

Typical reaction temperatures used for preferred solvents in reaction variation I are as follows:
n-hexane: 20 to 70 °C; n-heptane: 20 to 98 °C; i-octane: 20 to 99 °C; n-octane: 20 to 126 °C; cyclopentane: 20 to 50 °C; cyclohexane: 20 to 81 °C; cycloheptane: 20 to 118 °C; cyclooctane: 20 to 152 °C; petroleum ether: 20 to 70 °C; toluene: 20 to 110°C.

In method variation II the isomerization is carried out preferably at a temperature of 120 to 160 °C, more preferably at 140 to 160 °C.

The reaction time is preferably 1-5 hours, more preferably 1-3 hours.

Further preferred embodiments are defined in the dependent claims.

The shortest reaction time (2 hours) can be achieved by using a quaternary ammonium salt as phase transfer catalyst. The use of a fatty acid alkali metal salt also increases the reaction rate, but in a much lower degree. The reaction is slower (3 hours) in absence of phase transfer catalyst.

The soap formed in the reaction is treated with a mineral acid, and the product is obtained in the form of free fatty acid, which can be converted to esters, metal salts or glycerides. These steps can be accomplished by known methods.

For example, after the reaction the base can be neutralised and the fatty acid can be liberated from the soap by treatment with 10-20 volumes of aqueous mineral acid solution (hydrochloric acid, phosphoric acid, sulphuric acid) counted for one volume of hexane. The aqueous phase is washed several times with organic solvent to recover the residual fatty acids, then the combined organic phase is washed several times with water and finally with saturated sodium chloride solution. After drying over anhydrous drying agent, the organic solvent is removed under reduced pressure. The end product is saturated with an inert gas (for example nitrogen or argon) in order to avoid detrimental oxidation reactions.

In the isomerization method variation II according to the invention, the long chain alcohol (R-OH, wherein R represents a hydrocarbon chain of 6-20 carbon atoms) may be saturated or containing one unsaturation, it can be pure or a mixture of such alcohols. For example, n-octanol, n-decanol, n-dodecanol or the like, or their mixtures can be used as solvent. In the isomerization reaction the volume of the long chain alcohol is preferably 1-4 times greater than that of the oil, and preferably 1-5, more preferably 2-3,5 equivalents of the alkali metal hydroxide or the alkali earth metal hydroxide are used at a temperature of 100-170 °C. The reaction time is 0.5-10 hours, preferably 2-5 hours (especially 2 hours).

The product can be worked up in different ways using known methods. The long chain alcohol solvent can be removed by distillation without using any other organic solvent or water, or by extraction with a solvent, for example with hexane, or by chromatography, but these last two methods require great quantity of water and organic solvent. The long chain alcohol solvent can optionally be regenerated.

For example, during working up the reaction mixture is cooled, hexane is added, then the base is neutralised and the fatty acid is liberated from the soap with 2-5 volumes of aqueous concentrated mineral acid (hydrochloric acid, sulphuric acid, phosphoric acid) counted for one volume of the long chain alcohol. The mixture obtained is intensely stirred, and the aqueous and organic phases are separated. The aqueous phase is extracted with hexane once more, then the combined hexane phase is washed twice with water, once with saturated sodium chloride solution, and dried over anhydrous sodium sulphate. The hexane is evaporated under reduced pressure, and the long chain alcohol is removed by molecular distillation. The product is saturated with nitrogen in order to avoid detrimental oxidation.

In case of not using organic solvent extraction, the working up can be carried out as follows. To the reaction mixture cooled to 80-90 °C concentrated mineral acid solution (hydrochloric acid, phosphoric acid solution) is added in portions under intensive stirring. The salts precipitated are separated by centrifugation or filtered out, and then the long chain alcohols are removed by molecular distillation to avoid the oxidation of the product and the formation of undesired by-products.

In the methods according to the invention generally the quantity of isomerized linoleic acid is 50-80 %, and the quantity of the two main isomers (*c*9,*t*11 and *t*10,*c*12) is 48-78 %, depending on the fatty acid composition (linoleic acid content) of the starting material.

The methods according to the invention can be preferably used for the preparation of fatty acids or mixtures thereof containing high quantity of conjugated linoleic acid, wherein the ratio of the two biologically active isomers (*c*9,*t*11 and *t*10,*c*12) is approximately 1:1, and the total CLA content is between 50 and 100 % (depending on the linoleic acid content of the starting raw material). The isomerization of linoleic acid obtainable with the present method is of 95-100 %. Due to the method and mild reaction conditions (low temperature, short reaction time) used, the product, independently form the starting material, usually contains other (for example *t*,*t*-CLA, other *c*,*t*- and *t*,*c*-CLA, as well as *c*,*c*-CLA) isomers only in negligible (1-4 %) quantity. A further advantage of the methods that most of the solvents applicable in the reaction are accepted in the vegetable oil industry and can be easily regenerated.

A particular advantage of the method variation I according to the invention is that in spite of the lower temperature used in the apolar solvent, the isomerization is completed in a short reaction time. A conversion of at least 95 % can be achieved, in the product obtained mainly the two biologically active isomers can be found, and the undesired isomers are formed in small quantities. The solvent used can be easily regenerated and recycled. The method is especially suitable for the isomerization of free linoleic acid, where the end product is also free fatty acid. In case of using oil as starting material in the isomerization reaction, in addition to the free acid the product contains the tert.butyl ester of the fatty acid too, since in alkaline medium the triglycerid readily undergoes transesterification to t-butyl ester, which is stable in alkaline medium. The t-butyl ester can either be subjected to acidic hydrolysis or separated from the free acid and processed further.

An additional advantage of the method variation II according to the invention is that in the water immiscible long chain alcohol used as solvent, each of the starting material (vegetable oil, fatty acid, deodorization distillate), the product and the base used easily dissolve. Thus, also potassium hydroxide is suitable for the isomerization, which has a weaker base strength and inexpensive. Under the conditions of the reaction other products (for example ester) are formed only in small quantities (up to 3 %). Because of the high boiling point of the solvent the method does not require an explosion-proof equipment even in case of employing relatively higher temperature. A further advantage is that the reaction can be accomplished in a smaller quantity of solvent, and most of the solvent used can be recovered in pure form during the working up.

The details of the invention are demonstrated by the following non-limiting examples. The examples contain the detailed descriptions of certain preferable embodiments, only. It is obvious for a skilled person that different modifications can be made without departing from the spirit and scope of the invention.

### Example 1

Isomerization of fatty acid mixture of high linoleic acid content with potassium t-butylate in the presence of a phase transfer catalyst of quaternary ammonium salt type in n-hexane

Into a reactor equipped with a stirrer providing intense stirring and a reflux condenser 28 g (250 mmol) of t-BuOK, 0.14 g of phase transfer catalyst (TBACI, 1 % of the quantity of linoleic acid) and 500 ml of n-hexane were charged. While stirring, the mixture was warmed to about 50 °C, then 19 g of fatty acid mixture was added containing 75 % linoleic acid (14 g, 50 mmol of linoleic acid). The reaction time was 2 hours at 68-70 °C.

To the cooled reaction mixture 45 ml of concentrated hydrochloric acid in 250 ml of water was added to neutralise the base and to liberate the fatty acid from the soap. After intense stirring, the aqueous and organic phases were separated. The aqueous phase was extracted once more with 250 ml of hexane, then the combined hexane phase was washed with 2 x 500 ml of water and 1 x 500 ml of saturated sodium chloride solution. The hexane phase was dried over anhydrous sodium sulphate, then the hexane was removed under reduced pressure. The product was saturated with nitrogen to avoid detrimental oxidation.

The fatty acid composition of the product was determined by gas chromatography. The analytical conditions were as follows:
Equipment: Hewlett Packard 6890 GC
Injector: 100:1, 250 °C
Detector: FID detector at 250 °C
Carrier gas: H₂
Column: SGE BPX 70, 60 m x 0.22 mm, 0.25 µm
Oven program: from 150 °C to 210 °C at 1.3 °C/min heating rate

The fatty acid composition of the product is given in Table 1.

**Table 1**

| Fatty acid | % of weight |
|---|---|
| C16:0 | 7.01 |
| C16:1 | 0.08 |
| C18:0 | 2.85 |
| C18:1 | 14.72 |
| C18:2 trans (not conjugated) | 0.28 |
| *c*9,*t*12-C18:2 | 2.44 |
| *c*9,*t*11-CLA | 31.03 |
| *t*10,*c*12-CLA | 36.14 |
| *c*,*c*-CLA | 1.21 |
| *t*,*t*-CLA | 3.41 |
| C22:0 | 0.31 |
| C24:0 | 0.14 |
| C24:1 | 0.12 |
| Not identified | 0.26 |
| Total CLA | 71.79 |
| Main CLA isomers | 67.17 |
| Conversion | 96 % |

### Example 2

Isomerization of fatty acid mixture of high linoleic acid content with potassium t-butylate in the presence of phase transfer catalyst of alkali metal soap type in n-hexane

The method described in Example 1 was followed, but instead of quaternary ammonium salt potassium linoleate was used as phase transfer catalyst in 0.1 % quantity related to the amount of linoleic acid. The reaction time was 2.5 hours at 69-70 °C.

The fatty acid composition of the product was determined by gas chromatography. The analytical conditions were the same as described in Example 1. The fatty acid composition of the product is presented in Table 2.

**Table 2**

| Fatty acid | % by weight |
|---|---|
| C16:0 | 7.28 |
| C16:1 | - |
| C18:0 | 2.79 |
| C18:1 | 14.96 |
| C18:2 trans (not conjugated) | - |
| c9,c12-C18:2 | 1.53 |
| *c*9,*t*11-CLA | 33.58 |
| *t*10,*c*12-CLA | 39.00 |
| *c*,*c*-CLA | - |
| *t*,*t*-CLA | 0.86 |
| C22:0 | - |
| C24:0 | - |
| C24:1 | - |
| Not identified | - |
| Total CLA | 73.44 |
| Main CLA isomers | 72.58 |
| Conversion | 98% |

### Example 3

### Isomerization of fatty acid mixture of high linoleic acid content with potassium t-butylate in the absence of phrase transfer catalyst in n-hexane

The method described in Example 1 was followed, but in the absence of phase transfer catalyst. The reaction time was 3 hours at 69-70 °C.

The fatty acid composition of the product was determined by gas chromatography. The analytical conditions were the same as described in Example 1. The fatty acid composition of the product is presented in Table 3.

**Table 3**

| Fatty acid | % by weight |
|---|---|
| C16:0 | 6.95 |
| C16:1 | - |
| C18:0 | 2.47 |
| C18:1 | 14.46 |
| C18:2 trans (not conjugated) | - |
| *c*9,*c*12-C18:2 | 1.48 |
| *c*9,*t*11-CLA | 34.29 |
| *t*10,*c*12-CLA | 38.35 |
| *c*,*c*-CLA | 0.97 |
| *t*,*t*-CLA | 1.03 |
| C22:0 | - |
| C24:0 | - |
| C24:1 | - |
| Not identified | - |
| Total CLA | 74.64 |
| Main CLA isomers | 72.64 |
| Conversion | 98 % |

### Example 4

### Isomerization of fatty acid mixture of high linoleic acid content with potassium t-butylate in the absence of phase transfer catalyst in n-heptane

The method described in Example 1 was followed, but in the absence of phase transfer catalyst and n-heptane was used as solvent instead of n-hexane. The reaction time was 2 hours at 95-98 °C.

The fatty acid composition of the product was determined by gas chromatography. The analytical conditions were the same as given in Example 1. The fatty acid composition of the product is presented in Table 4.

**Table 4**

| Fatty acid | % by weight |
|---|---|
| C16:0 | 6.92 |
| C16:1 | - |
| C18:0 | 2.48 |
| C18:1 | 14.51 |
| C18:2 trans (not conjugated) | - |
| *c*9,*c*12-C18:2 | 0.77 |
| *c*9,*t*11-CLA | 34.49 |
| *t*10,*c*12-CLA | 38.86 |
| *c*,*c*-CLA | 0.97 |
| *t*,*t*-CLA | 1.00 |
| C22:0 | - |
| C24:0 | - |
| C24:1 | - |
| Not identified | - |
| Total CLA | 75.32 |
| Main CLA isomers | 73.35 |
| Conversion | 99 % |

### Example 5

### Isomerization of vegetable oil of high linoleic acid content with alkali metal alcoholate in the presence of phase transfer catalyst of quaternary ammonium salt type at low temperature

Into a reactor equipped with a stirrer providing intense stirring and a reflux condenser 28 g (250 mmol) of t-BuOK, 0.14 g of phase transfer catalyst (TBACI, 1 % of the quantity of linoleic acid) and 600 ml of n-hexane were charged. The stirred mixture was warmed to about 50 °C, then 21 g of safflower oil was added containing 75 % linoleic acid (14 g, 50 mmol of linoleic acid). The reaction time was 2 hours at 68-70 °C.

The reaction mixture was worked up as described in Example 1. Two products were formed: free fatty acid and fatty acid t-butyl ester. The two fractions were separated by preparative column chromatography using hexane - acetone mixture (10:0.05) as eluent. The ratio of free fatty acid to t-butyl ester was 3:1.

The fatty acid composition of the product was determined by gas chromatography. The analytical conditions were the same as described in Example 1. The fatty acid composition of the product is presented in Table 5.

**Table 5**

| Fatty acid | % by weight Free fatty acid fraction | t-Butyl ester fraction |
|---|---|---|
| C16:0 | 7.56 | 7.57 |
| C16:1 | 0.09 | - |
| C18:0 | 2.45 | 2.40 |
| C18:1 | 14.22 | 14.31 |
| C18:2 trans (not conjugated) | 0.17 | - |
| *c*9,*c*12-C18:2 | 0.27 | - |
| *c*9,*t*11-CLA | 33.39 | 35.62 |
| *t*10,*c*12-CLA | 37.98 | 38.01 |
| *c,c*-CLA | 1.10 | 0.39 |
| *t*,*t*-CLA | 1.73 | 1.28 |
| C22:0 | 0.27 | - |
| C24:0 | 0.13 | - |
| C24:1 | - | - |
| Not identified | 0.65 | - |
| Total CLA | 74.20 | 75.30 |
| Main CLA isomers | 71.37 | 73.63 |
| Conversion | 99 % | |

### Example 6

### Isomerization of fatty acid mixture of high linoleic acid content with potassium t-butylate in the absence of phase transfer catalyst in n-hexane

The method described in Example 1 was followed, but in the absence of phase transfer catalyst and the reaction was carried out at 50 °C. The reaction time was 8 hours at 50 °C.

The fatty acid composition of the product was determined by gas chromatography. The analytical conditions were the same as described in Example 1. The fatty acid composition of the product is presented in Table 6.

**Table 6**

| Fatty acid | % by weight |
|---|---|
| C16:0 | 7.65 |
| C16:1 | 0.13 |
| C18:0 | 2.72 |
| C18:1 | 14.64 |
| C18:2 trans (not conjugated) | 2.03 |
| *c*9,*c*12-C18:2 | 33.29 |
| *c*9,*t*11-CLA | 17.27 |
| *t*10,*c*12-CLA | 20.95 |
| *c*,*c*-CLA | 0.52 |
| *t*,*t*-CLA | 0.53 |
| C22:0 | 0.27 |
| C24:0 | - |
| C24:1 | - |
| Not identified | - |
| Total CLA | 39.27 |
| Main CLA isomers | 38.22 |
| Conversion | 53 % |

### Example 7

### Isomerization of fatty acid mixture of high linoleic acid content with potassium t-butylate in toluene

Into a round bottom flask equipped with a reflux condenser 1.122 g (10 mmol) of t-BuOK and 30 ml of toluene were charged. Intensive stirring was provided by magnetic stirrer. While stirring, the mixture was warmed to about 50 °C, then 0.75 g of fatty acid mixture was added containing 75 % linoleic acid (0.56 g, 2 mmol of linoleic acid). The reaction time was 1 hour at 68-72 °C.

To the cooled reaction mixture 5 ml of concentrated hydrochloric acid in 15 ml of water was added to neutralise the base and to liberate the fatty acid from the soap. After intense stirring, the aqueous and organic phases were separated. The aqueous phase was extracted once more with 50 ml of hexane, then the combined organic phase was washed with 2 x 50 ml of water and 1 x 50 ml of saturated sodium chloride solution. The organic phase was dried over anhydrous sodium sulphate, then the solvent was removed under reduced pressure. The product was saturated with nitrogen to avoid detrimental oxidation.

The fatty acid composition of the product was determined by gas chromatography. The analytical conditions were the same as described in Example 1. The fatty acid composition of the product is presented in Table 7.

**Table 7**

| Fatty acid | % by weight |
|---|---|
| C16:0 | 7.59 |
| C16:1 | - |
| C18:0 | 2.62 |
| C18:1 | 14.62 |
| C18:2 trans (not conjugated) | 0.56 |
| *c*9,*c*12-C18:2 | 0.11 |
| *c*9,*t*11-CLA | 35.97 |
| *t*10,*c*12-CLA | 36.17 |
| *c*,*c*-CLA | 0.98 |
| *t*,*t*-CLA | 1.38 |
| C22:0 | - |
| C24:0 | - |
| C24:1 | - |
| Not identified | - |
| Total CLA | 74.50 |
| Main CLA isomers | 72.14 |
| Conversion | 99 % |

### Example 8

### Isomerization of fatty acid mixture of high linoleic acid content with potassium t-butylate in toluene

Into a round bottom flask equipped with a reflux condenser 0.56 g (5 mmol) of t-BuOK and 30 ml of toluene were charged. Intensive stirring was provided by magnetic stirrer. While stirring, the mixture was warmed to about 50 °C, then 0.75 g of fatty acid mixture was added containing 75 % linoleic acid (0.56 g, 2 mmol of linoleic acid). The reaction time was 1 hour at 110 °C.

To the cooled reaction mixture 5 ml of concentrated hydrochloric acid in 1 5 ml of water was added to neutralise the base and to liberate the fatty acid from the soap. After intense stirring, the aqueous and organic phases were separated. The aqueous phase was extracted once more with 50 ml of hexane, then the combined organic phase was washed with 2 x 50 ml of water and 1 x 50 ml of saturated sodium chloride solution. The organic phase was dried over anhydrous sodium sulphate, then the solvent was removed under reduced pressure. The product was saturated with nitrogen to avoid detrimental oxidation.

The fatty acid composition of the product was determined by gas chromatography. The analytical conditions were the same as described in Example 1. The fatty acid composition of the product is presented in Table 8.

**Table 8**

| Fatty acid | % by weight |
|---|---|
| C16:0 | 7.62 |
| C16:1 | - |
| C18:0 | 2.61 |
| C18:1 | 14.68 |
| C18:2 trans (not conjugated) | - |
| *c*9,*c*12-C18:2 | - |
| *c*9,*t*11-CLA | 37.20 |
| *t*10,*c*12-CLA | 34.34 |
| *c*,*c*-CLA | 1.34 |
| *t*,*t*-CLA | 2.21 |
| C22:0 | - |
| C24:0 | - |
| C24:1 | - |
| Not identified | - |
| Total CLA | 75.09 |
| Main CLA isomers | 71.54 |
| Conversion | 100 % |

### Example 9

### Isomerization of fatty acid mixture of high linoleic acid content with potassium hydroxide

In a reactor equipped with stirrer providing intense stirring and a thermometer 14 g (250 mmol) of potassium hydroxide was dissolved in 50 ml of octanol, then 19 g of fatty acid mixture containing 75 % of linoleic acid (14 g, 50 mmol of linoleic acid) was added to the solution. The reaction time was 2 hours at 150 °C.

After cooling 150 ml of hexane and 45 ml of concentrated hydrochloric acid in 250 ml of water was added to the reaction mixture. The phases were separated, the aqueous phase was washed with 1 x 200 ml of hexane, then the combined hexane phase was washed with 3 x 250 ml of water and 1 x 250 ml of saturated sodium chloride solution. The organic phase was dried over anhydrous sodium sulphate, the hexane was evaporated under reduced pressure and the octanol was removed by molecular distillation (160 °C, 5 mbar).

The fatty acid composition of the product was determined by gas chromatography. The analytical conditions were the same as described in Example 1. The fatty acid composition of the product is summarised in Table 9.

**Table 9**

| Fatty acid | % by weight |
|---|---|
| C16:0 | 8.11 |
| C16:1 | - |
| C18:0 | 2.82 |
| C18:1 | 14.26 |
| C18:2 trans (not conjugated) | - |
| *c*9,*c*12-C18:2 | 1.52 |
| *c*9,*t*11-CLA | 35.54 |
| *t*10,*c*12-CLA | 35.60 |
| c, c-C LA | 1.63 |
| *t*,*t*-CLA | 0.52 |
| C22:0 | - |
| C24:0 | - |
| C24:1 | - |
| Not identified | - |
| Total CLA | 73.29 |
| Main CLA isomers | 71.14 |
| Conversion | 98 % |

### Example 10

### Isomerization of safflower oil with potassium hydroxide in octanol

In a reactor providing intense stirring and equipped with thermometer 2 kg of potassium hydroxide (36 mol) was dissolved in 10 I of octanol, then 5 I of safflower oil containing 75 % of linoleic acid (4 kg, 16 mol of linoleic acid) was added to the solution. The reaction time was 3 hours at 160 °C.

To the stirred reaction mixture cooled to 90 °C 2.4 I of 75 % phosphoric acid solution was added. The salts formed from the base (potassium phosphates) were separated by centrifugation (25 minutes, 5000 rpm). The upper phase was washed with 1 x 2 I of water under intense stirring, then it was centrifuged again (25 minutes, 5000 rpm).

From the octanol solution the solvent was removed by molecular distillation in two steps. First about 95 % of the octanol was distilled off at lower temperature, at 130 °C, under lower vacuum (8-10 mbar), then the rest of the octanol was removed at higher temperature (180-190 °C) and under lower pressure (1-3 mbar).

The fatty acid composition of the product was determined by gas chromatography. The analytical conditions were the same as described in Example 1. The fatty acid composition of the product is summarised in Table 10.

**Table 10**

| Fatty acid | % by weight |
|---|---|
| C16:0 | 7.77 |
| C16:1 | - |
| C18:0 | 2.45 |
| C18:1 | 14.41 |
| C18:2 trans (not conjugated) | 0.55 |
| c9,c12-C18:2 | 0.27 |
| *c*9,*t*11-CLA | 35.92 |
| *t*10,*c*12-CLA | 33.31 |
| *c*,*c*-CLA | 1.88 |
| *t*,*t*-CLA | 1.72 |
| C22:0 | - |
| C24:0 | - |
| C24:1 | - |
| Not identified | 1.72 |
| Total CLA | 72.83 |
| Main CLA isomers | 69.23 |
| Conversion | 97 % |

### Example 11

### Isomerization of a deodorization distillate with potassium hydroxide in octanol

In a reactor providing intense strirring and equipped with thermometer 120 g (2.1 mol) of potassium hydroxide was dissolved in 500 ml of octanol at 150 °C, then under stirring 500 ml of deodorization distillate containing 41 % of linoleic acid (170 g, 0.6 mol linoleic acid) was added to the solution. The reaction time was 3 hours at 160 °C.

To the stirred reaction mixture cooled to 90 °C 100 ml of 75 % phosphoric acid solution was added. The salts formed from the base (potassium phosphates) were separated by centrifugation (25 minutes, 5000 rpm). The upper phase was washed 1 x 1 I of water under intense stirring, then it was centrifuged again (25 minutes, 5000 rpm).

From the octanol solution the solvent was removed by molecular distillation in two steps. First about 95 % of the octanol was distilled off at lower temperature, at 130 °C, under lower vacuum (8-10 mbar), then the rest of the octanol was removed at higher temperature (180-190 °C) and under lower pressure (1-3 mbar).

The fatty acid composition of the product was determined by gas chromatography. The analytical conditions were the same as described in Example 1. The fatty acid composition of the product is summarised in Table 11.

**Table 11**

| Fatty acid | % by weight |
|---|---|
| C16:0 | 9.38 |
| C16:1 | - |
| C18:0 | 4.88 |
| C18:1 | 24.37 |
| C18:2 trans (not conjugated) | - |
| *c*9,*c*12-C18:2 | 0.86 |
| *c*9,*t*11-CLA | 28.35 |
| *t*10,*c*12-CLA | 28.48 |
| *c*,*c*-CLA | 1.51 |
| *t*,*t*-CLA | 0.95 |
| C22:0 | - |
| C24:0 | - |
| C24:1 | - |
| Not identified | 1.22 |
| Total CLA | 59.29 |
| Main CLA isomers | 56.83 |
| Conversion | 99 % |

### Example 12

### Isomerization of fatty acid mixture of high linoleic acid content with potassium t-butylate

In a reactor providing intense stirring and equipped with thermometer 11.22 g (100 mmol) of potassium t-butylate was dissolved in 50 ml of octanol under stirring at 150 °C, then 7.5 g of fatty acid mixture containing 75 % of linoleic acid (5.6 g, 20 mmol of linoleic acid) was added to the solution. The reaction time was 2 hours at 150 °C.

To the cooled mixture 150 ml of hexane and 45 ml of concentrated hydrochloric acid in 250 ml of water were added. The phases were separated, the aqueous phase was washed with 1 x 200 ml of hexane, then the combined hexane phases were washed with 3 x 250 ml of water and 1 x 250 ml of saturated sodium chloride solution. The organic phase was dried over anhydrous sodium sulphate, then the hexane was distilled off under reduced pressure, and the octanol was removed by molecular distillation (160 °C, 5 mbar).

The fatty acid composition of the product was determined by gas chromatography. The analytical conditions were the same as described in Example 1. The fatty acid composition of the product is summarised in Table 12.

**Table 12**

| Fatty acid | % by weight |
|---|---|
| C16:0 | 7.40 |
| C16:1 | - |
| C18:0 | 2.47 |
| C18:1 | 14.10 |
| C18:2 trans (not conjugated) | 0.51 |
| *c*9,*c*12-C18:2 | 0.23 |
| *c*9,*t*11-CLA | 34.55 |
| *t*10,*c*12-CLA | 33.22 |
| *c*,*c*-CLA | 1.94 |
| *t*,*t*-CLA | 4.43 |
| C22:0 | 0.25 |
| C24:0 | - |
| C24:1 | - |
| Not identified | 0.90 |
| Total CLA | 74.14 |
| Main CLA isomers | 67.77 |
| Conversion | 99 % |

### Example 13

### Isomerization of safflower oil with potassium hydroxide in octanol

In a reactor providing intense stirring and equipped with thermometer 14 g of potassium hydroxide (250 mmol) was dissolved in 50 ml of octanol, then 19 g of safflower oil containing 75 % of linoleic acid (14 g, 50 mmol of linoleic acid) was added to the solution. The reaction time was 8 hours at 120 °C.

To the cooled reaction mixture 150 ml of hexane and 45 ml of concentrated hydrochloric acid in 250 ml of water were added. The phases were separated, the aqueous phase was washed with 1 x 200 ml of hexane, then the combined organic phase was washed with 3 x 250 ml of water and 1 x 250 ml of saturated sodium chloride solution. The organic phase was dried over anhydrous sodium sulphate, the hexane was evaporated under reduced pressure and the octanol was removed by molecular distillation (160 °C, 5 mbar).

The fatty acid composition of the product was determined by gas chromatography. The analytical conditions were the same as described in Example 1. The fatty acid composition of the product is summarised in Table 13.

**Table 13**

| Fatty acid | % by weight |
|---|---|
| C16:0 | 7.45 |
| C16:1 | - |
| C18:0 | 2.39 |
| C18:1 | 13.94 |
| C18:2 trans (not conjugated) | 1.47 |
| *c*9,*c*12-C18:2 cis | 8.04 |
| *c*9,*t*11-CLA | 32.42 |
| *t*10,*c*12-CLA | 32.01 |
| *c*,*c*-CLA | 1.17 |
| *t*,*t*-CLA | 0.64 |
| C22:0 | 0.23 |
| C24:0 | - |
| C24:1 | - |
| Not identified | 0.24 |
| Total CLA | 66.24 |
| Main CLA isomers | 64.43 |
| Conversion | 87 % |

### Example 14

### Isomerization of safflower oil with potassium hydroxide in octanol

In a reactor providing intense stirring and equipped with thermometer 7 g of potassium hydroxide (125 mmol) was dissolved in 50 ml of octanol, then 19 g of safflower oil containing 75 % of linoleic acid (14 g, 50 mmol of linoleic acid) was added to the solution. The reaction time was 24 hours at 120 °C.

To the cooled reaction mixture 150 ml of hexane and 30 ml of concentrated hydrochloric acid in 250 ml of water were added. The phases were separated, the aqueous phase was washed with 1 x 200 ml of hexane, then the combined organic phase was washed with 3 x 250 ml of water and 1 x 250 ml of saturated sodium chloride solution. The organic phase was dried over anhydrous sodium sulphate, the hexane was evaporated under reduced pressure and the octanol was removed by molecular distillation (160 °C, 5 mbar).

The fatty acid composition of the product was determined by gas chromatography. The analytical conditions were the same as described in Example 1. The fatty acid composition of the product is summarised in Table 14.

**Table 14**

| Fatty acid | % by weight |
|---|---|
| C16:0 | 7.22 |
| C16:1 | 0.11 |
| C18:0 | 2.38 |
| C18:1 | 13.93 |
| C18:2 trans (not conjugated) | 1.90 |
| c9,c12-C18:2 cis | 43.95 |
| *c*9,*t*11-CLA | 15.23 |
| *t*10,*c*12-CLA | 14.07 |
| *c*,*c*-CLA | 0.64 |
| *t*,*t*-CLA | 0.32 |
| C22:0 | 0.25 |
| C24:0 | - |
| C24:1 | - |
| Not identified | - |
| Total CLA | 30.26 |
| Main CLA isomers | 29.30 |
| Conversion | 40 % |

## Claims

1. A method for the preparation of conjugated linoleic acid from at least one linoleic acid-containing starting material by alkali isomerization, comprising treating the at least one linoleic acid-containing starting material with a base, wherein as solvent an apolar aprotic solvent or a mixture of apolar aprotic solvents is used, as base an alkali metal alcoholate is used and the quantity of the base used is 1-10 moles per one mole of linoleic acid, preferably 5 moles per one mole of linoleic acid.

2. The method according to claim 1, **characterised in that** the isomerization is carried out at a temperature ranging from 20°C to the boiling point of the solvent.

3. The method according to claim 1 or 2, **characterised in that** the at least one linoleic acid-containing starting material is a material of high linoleic acid content.

4. The method according to claim 3, **characterised in that** the material of high linoleic acid content is selected from the group consisting of
a) oils, preferably sunflower oil, safflower oil, soybean oil, com oil and mixtures of oils,
b) fatty acid mixtures obtainable from oils, preferably from sunflower oil, safflower oil, soybean oil, com oil,
c) pure linoleic acid,
d) by-products being formed during refining of vegetable oils, preferably the deodorization distillate,
and mixtures containing two or more materials of those listed under a), b), c) and d) in any ratio.

5. The method according to any of claims 1 to 4, **characterised in that** the reaction time is 1-3 hours.

6. The method according to any of claims 1 to 5, **characterised in that** the alcohol component of the alcoholate is a tertiary alcohol, preferably t-butyl alcohol.

7. The method according to any of claims 1 to 6, **characterised in that** the alkali metal component of the alcoholate is potassium.

8. The method according to any of claims 1 to 7, **characterised in that** the alcoholate is potassium t-butylate.

9. The method according to any of claims 1 to 8, **characterised in that** the isomerization is carried out in presence of a phase transfer catalyst.

10. The method according to claim 9, **characterised in that** the phase transfer catalyst is a compound of quaternary ammonium salt type, preferably tetrabutylammonium hydrogensulphate (TBAHSO₄) or tetrabutylammonium chloride (TBACI).

11. The method according to claim 9, **characterised in that** the phase transfer catalyst is a compound of alkali metal soap type, preferably potassium linoleate.

12. The method according to any of claims 1 to 11, **characterised in that** the aprotic apolar solvent is an acyclic or cyclic alkane, preferably n-hexane.

13. The method according to any of claims 1 to 12, **characterised in that** the isomerization is carried out at a temperature ranging from 55°C to the boiling point of the solvent.

14. The method according to any of claims 1 to 13, **characterised in that** the isomerization is carried out at 55-70 °C.

15. A method for the preparation of conjugated linoleic acid from at least one linoleic acid-containing starting material by alkali isomerization, comprising treating the at least one linoleic acid-containing starting material with a base, wherein as solvent a long chain alcohol containing 6-20 carbon atoms or a mixture of long chain alcohols containing 6-20 carbon atoms is used, as base an alkali metal hydroxide or an alkali earth metal hydroxide or an alkali metal alcoholate is used, and the isomerization is carried out at 100-170 °C.

16. The method according to claim 15, **characterised in that** the at least one linoleic acid-containing starting material is a material of high linoleic acid content.

17. The method according to claim 16, **characterised in that** the material of high linoleic acid content is selected from the group consisting of
a) oils, preferably sunflower oil, safflower oil, soybean oil, com oil and mixtures of oils,
b) fatty acid mixtures obtainable from oils, preferably from sunflower oil, safflower oil, soybean oil, com oil,
c) pure linoleic acid,
d) by-products being formed during refining of vegetable oils, preferably the deodorization distillate,
and mixtures containing two or more materials of those listed under a), b), c) and d) in any ratio.

18. The method according to any of claims 15 to 17, **characterised in that** the reaction time is 2-3 hours.

19. The method according to any of claims 15 to 18, **characterised in that** as base potassium hydroxide is used.

20. The method according to any of claims 15 to 19, **characterised in that** as solvent n-octanol is used.

21. The method according to any of claims 15 to 20, **characterised in that** the isomerization is carried out at 120-160 °C, preferably at 140-160 °C.

22. The method according to any of claims 15 to 21, **characterised in that** the quantity of the base used is 1-5 mol equivalents, preferably 2-3.5 mol equivalents related to the quantity of linoleic acid.

## Patentansprüche

1. Verfahren zur Herstellung von konjugierter Linolsäure aus mindestens einem, Linolsäure enthaltenden Ausgangsmaterial, durch Alkaliisomerisierung, wobei das mindestens ein Linolsäure enthaltendes Ausgangsmaterial mit einer Base behandelt wird, wobei als Lösungsmittel ein apolares protonenfreies Lösungsmittel oder eine Mischung von apolaren protonenfreien Lösungsmitteln und als Base ein Alkalimetallalkoholat verwendet wird, und wobei die Menge der verwendeten Base ist 1-10 Mol Base pro 1 Mol Linolsäure, vorzugsweise 5 Mol Base pro 1 Mol Linolsäure.

2. Verfahren nach Anspruch 1, **dadurch gekennzeichnet, dass** die Isomerisierung bei einer Temperatur von zwischen 20 °C und dem Siedepunkt des Lösungsmittels ausgeführt wird.

3. Verfahren nach Ansprüche 1 oder 2, **dadurch gekennzeichnet, dass** das mindestens ein Linolsäure enthaltendes Ausgangsmaterial ein Material mit hohem Linolsäure-Anhalt ist.

4. Verfahren nach Anspruch 3, **dadurch gekennzeichnet, dass** das mindestens ein Linolsäure enthaltendes Ausgangsmaterial aus der folgender Gruppe ausgewählt wird:
a) Öle, vorzugsweise Sonnenblumenöl, Distelöl, Sojaöl, Maiskeimöl und Mischungen von Ölen,
b) aus Ölen, vorzugsweise aus Sonnenblumenöl, Distelöl, Sojaöl und Maiskeimöl herstellbare Fettsäuremischungen,
c) reine Linolsäure,
d) während der Raffinierung von Pflanzenölen entstehende Nebenprodukte, vorzugsweise das Desodorierungsdestillat,
und Mischungen aus zwei oder mehreren Materialen angegeben in a)-d) in beliebigem Verhältnis.

5. Verfahren nach einem der Ansprüche 1-4, **dadurch gekennzeichnet, dass** die Reaktionszeit 1-3 Stunden beträgt.

6. Verfahren nach einem der Ansprüche 1-5, **dadurch gekennzeichnet, dass** die Alkoholkomponente des Alkoholates ein tertiärer Alkohol, vorzugsweise t-Butylalkohol ist.

7. Verfahren nach einem der Ansprüche 1-6, **dadurch gekennzeichnet, dass** die Alkalimetallkomponente des Alkoholates Kalium ist.

8. Verfahren nach einem der Ansprüche 1-7, **dadurch gekennzeichnet, dass** das Alkoholat Kalium-t-butylat ist.

9. Verfahren nach einem der Ansprüche 1-8, **dadurch gekennzeichnet, dass** die Isomerisierung in der Anwesenheit eines Phasenübertragungskatalysators ausgeführt wird.

10. Verfahren nach Anspruch 9, **dadurch gekennzeichnet, dass** der Phasenübertragungskatalysator ein Verbindung von quartärem Ammoniumsalz-Typ, vorzugsweise Tetrabutylammoniumhydrogensufat (TBAHSO₄) oder Tertrabutylammoniumchlorid (TBACI) ist.

11. Verfahren nach Anspruch 9, **dadurch gekennzeichnet, dass** der Phasenübertragungskatalysator ein Verbindung von Alkalimetallseife-Typ, vorzugsweise Kaliumlinoleat ist.

12. Verfahren nach einem der Ansprüche 1-11, **dadurch gekennzeichnet, dass** das protonenfreies apolares Lösungsmittel ein acyklisches oder cyklisches Alkan, vorzugsweise n-Hexan ist.

13. Verfahren nach einem der Ansprüche 1-12, **dadurch gekennzeichnet, dass** die Isomerisierung bei einer Temperatur von zwischen 55 °C und dem Siedepunkt des Lösungsmittels ausgeführt wird.

14. Verfahren nach einem der Ansprüche 1-12, **dadurch gekennzeichnet, dass** die Isomerisierung bei einer Temperatur von 55-70 °C ausgeführt wird.

15. Verfahren zur Herstellung von konjugierter Linolsäure aus mindestens einem, Linolsäure enthaltenden Ausgangsmaterial, durch Alkaliisomerisierung, wobei das mindestens ein Linolsäure enthaltendes Ausgangsmaterial mit einer Base behandelt wird, wobei als Lösungsmittel ein langkettiger Alkohol mit 6-20 Kohlenstoffatome oder eine Mischung von langkettigen Alkoholen mit 6-20 Kohlenstoffatome und als Base ein Alkalimetallhydroxid oder ein Erdalkalimetallhydroxid oder ein Alkalimetallalkoholat verwendet wird, und wobei die Isomerisation bei einer Temperatur von 100-170 °C ausgeführt wird.

16. Verfahren nach Anspruch 15, **dadurch gekennzeichnet, dass** das mindestens ein Linolsäure enthaltendes Ausgangsmaterial ein Material mit hohem Linolsäure-Anhalt ist.

17. Verfahren nach Anspruch 16, **dadurch gekennzeichnet, dass** das mindestens ein Linolsäure enthaltendes Ausgangsmaterial aus der folgender Gruppe ausgewählt wird:
a) Öle, vorzugsweise Sonnenblumenöl, Distelöl, Sojaöl, Maiskeimöl und Mischungen von Ölen,
b) aus Ölen, vorzugsweise aus Sonnenblumenöl, Distelöl, Sojaöl und Maiskeimöl herstellbare Fettsäuremischungen,
c) reine Linolsäure,
d) während der Raffinierung von Pflanzenölen entstehende Nebenprodukte, vorzugsweise das Desodorierungsdestillat,
und Mischungen aus zwei oder mehreren Materialen angegeben in a)-d) in beliebigem Verhältnis.

18. Verfahren nach einem der Ansprüche 15-17, **dadurch gekennzeichnet, dass** die Reaktionszeit 2-3 Stunden beträgt.

19. Verfahren nach einem der Ansprüche 15-18, **dadurch gekennzeichnet, dass** als Base Kaliumhydroxid verwendet wird.

20. Verfahren nach einem der Ansprüche 15-19, **dadurch gekennzeichnet, dass** als Lösungsmittel n-Octanol verwendet wird.

21. Verfahren nach einem der Ansprüche 15-20, **dadurch gekennzeichnet, dass** die Isomerisierung bei einer Temperatur von 120-160 °C, vorzugsweise 140-160 °C ausgeführt wird.

22. Verfahren nach einem der Ansprüche 15-20, **dadurch gekennzeichnet, dass** die Menge der Base beträgt 1-5 Mol-Äquivalent, vorzugsweise 2-3,5 Mol-Äquivalent bezüglich die Menge der Linolsäure.

## Revendications

1. Procédé pour la préparation d'acide linoléique conjugué à partir au moins d'une matière première contenant d'acide linoléique réalisé par une isomérisation alcaline, dans laquelle au moins une matière première contenant d'acide linoléique est soumise à un traitement avec une base, dans lequel un solvant apolaire aprotique ou un mélange des solvants apolaires aprotiques sont utilisés comme solvant, un alcoolate d'un métal alcalin est utilisé comme base et la base est utilisée en quantité entre 1-10 en mol éqiuvalents par rapport à une mole de l'acide linoléique, en préférence en quantité de 5 en mol équivalents par rapport à une mole de l'acide linoléique.

2. Procédé selon la revendication 1, **caractérisé en ce que** l'isomérisation est réalisée à une temperature variant entre 20 °C et la température d'ébullition de solvant.

3. Procédé selon la revendication 1 ou 2, **caractérisé en ce qu'**au moins une matière première contenant d'acide linoléique est une matière à haute teneur en acide linoléique.

4. Procédé selon la revendication 3, **caractérisé en ce que** la matière à haute teneur en acide linoléique est choisie dans le groupe formé par
a) les huiles, en préférence l'huile de tournesol, l'huile de carthame, l'huile de soja, l'huile de maïs and les mélanges des celles-ci;
b) mélanges des acides gras accessibles à partir de l'huils, en préférence de l'huile de tournesol, l'huile de carthame, l'huile de soja, l'huile de maïs;
c) l'acide linoléique pur;
d) les coproduits créés ou cours du raffinage des huiles végetales, en préférence le distillat de désodorisation;
et les mélanges contenant en n'importe quelle proportion deux ou plusieurs matières énumérées sous a), b), c) et d).

5. Procédé selon l'une quelconque des revendications 1 à 4 **caractérisé en ce que** la durée de la réaction est entre 1-3 heures.

6. Procédé selon l'une quelconque des revendications 1 à 5 **caractérisé en ce que** le component d'alcool de l'alcoolate est un alcool tertiaire, en préférence l'alcool butylique tertiaire.

7. Procédé selon l'une quelconque des revendications 1 à 6 **caractérisé en ce que** le component de métal alcalin de l'alcoolate est le potassium.

8. Procédé selon l'une quelconque des revendications 1 à 7 **caractérisé en ce que** l'alcoolate est le butylate tertiaire de potassium.

9. Procédé selon l'une quelconque des revendications 1 à 8 **caractérisé en ce que** l'isomerisation est réalisée en présence d'un catalyseur de transfert de phase.

10. Procédé selon la revendication 9 **caractérisé en ce que** le catalyseur de transfert de phase est un composée de type d'un sel d'ammonium quaternaire, en préference l'hydrogène sulphate de tetrabutylammonium (TBAHSO₄) ou le chlorure de tetrabutylammonium (TBACI).

11. Procédé selon la revendication 9, **caractérisé en ce que** le catalyseur de transfert de phase est un composé de type de savon d'un metal alcalin, en préférence linéolate de potassium.

12. Procédé selon l'une quelconque des revendications 1 à 11 **caractérisé en ce que** le solvant apolaire aprotique est un alcane non cyclique ou cyclique, en préférence n-hexane.

13. Procédé selon l'une quelconque des revendications 1 à 12 **caractérisé en ce que** l'isomerisation est réalisée à une temperature variant entre 55 °C et la température d'ébullition de solvant.

14. Procédé selon l'une quelconque des revendications 1 à 13 **caractérisé en ce que** l'isomerisation est réalisée à une temperature variant entre 55 °C et 70 °C.

15. Procédé pour la préparation d'acide linoléique conjugué à partir au moins une matière première contenant d'acide linoléique réalisé par une isomérisation alcaline, dans laquelle au moins une matière première contenant d'acide linoléique est soumise à un traitement avec une base, dans lequel un alcool à chaîne longue possédant 6-20 atomes de carbone ou un mélange des alcools à chaîne longue possédant 6-20 atomes de carbone sont utilisés comme solvant, un hydroxide de métal alcalin ou un hydroxide de métal alcalino-terreux ou un alcoolate de métal alcalin sont utilisés comme base, et l'isomerisation est réalisée à une temperature variant entre 100-170 °C.

16. Procédé selon la revendication 15, **caractérisé en ce qu'**au moins une matière première contenant d'acide linoléique est une matière à haute teneur en acide linoléique.

17. Procédé selon la revendication 16, **caractérisé en ce que** la matière à haute teneur en acide linoléique est choisie dans le groupe formé par
a) les huiles, en préférence l'huile de tournesol, l'huile de carthame, l'huile de soja, l'huile de maïs and les mélanges des celles-ci;
b) mélanges des acides gras accessibles à partir de l'huils, en préférence de l'huile de tournesol, l'huile de carthame, l'huile de soja, l'huile de maïs;
c) l'acide linoléique pur;
d) les coproduits créés ou cours du raffinage des huiles végetales, en préférence le distillat de désodorisation;
et les mélanges contenant en n'importe quelle proportion deux ou plusieurs matières énumérées sous a), b), c) et d).

18. Procédé selon l'une quelconque des revendications 15 à 17 **caractérisé en ce que** la durée de la réaction est entre 2-3 heures.

19. Procédé selon l'une quelconque des revendications 15 à 18 **caractérisé en ce que** l'hydroxide de potassium est utilisé comme base.

20. Procédé selon l'une quelconque des revendications 15 à 19 **caractérisé en ce que** n-octanol est utilisé comme solvant.

21. Procédé selon l'une quelconque des revendications 15 à 20 **caractérisé en ce que** l'isomérisation est réalisée à une temperature variant entre 120-160 °C, en préférence entre 140-160 °C.

22. Procédé selon l'une quelconque des revendications 15 à 21 **caractérisé en ce que** la base est utilisée en quantité entre 1-5 en mol éqiuvalents, en préférence en quantité entre 2-3,5 en mol équivalents par rapport à la quantité de l'acide linoléique.
